# FASCICULE DE BREVET EUROPEEN

(11) **EP 3 862 489 B1**
(45) Date de publication et mention de la délivrance du brevet: **28.12.2022**
(21) Numéro de dépôt: 21155184.1
(22) Date de dépôt: 04.02.2021
(51) Int. Cl.: E02D 1/02

(54) **DISPOSITIF ET SYSTÈME DE MESURE POUR LA CARACTÉRISATION GÉOMÉCANIQUE D'UN SOL, AINSI QUE PROCÉDÉ DE MESURE CORRESPONDANT**
MESSVORRCHTUNG UND -SYSTEM ZUR GEOMECHANISCHEN CHARAKTERISIERUNG EINES BODENS SOWIE ENTSPRECHENDES MESSVERFAHREN
MEASURING DEVICE AND SYSTEM FOR CHARACTERISING THE GEOMECHANICS OF SOIL, AND CORRESPONDING MEASUREMENT METHOD

(30) Priorité: 05.02.2020 FR 2001137
(43) Date de publication de la demande: 11.08.2021
(73) Titulaire: Sol Solution, 63200 Riom (FR)
(72) Inventeur: BENZ NAVARRETE, Miguel, 63100 CLERMONT-FERRAND (FR); BARBIER, Sébastien, 63000 CLERMONT FERRAND (FR)
(74) Mandataire: Lavoix

(56) Documents cités:
- JP-A- H06 317 510
- JP-A- H10 266 178
- JP-A- 2018 091 028
- US-A1- 2007 131 025

## Description

La présente invention concerne un dispositif de mesure pour la caractérisation géomécanique d'un sol. L'invention concerne également un système de mesure pour la caractérisation géomécanique d'un sol, comportant un tel dispositif de mesure. Elle concerne aussi un procédé de mesure pour la caractérisation géomécanique d'un sol.

En génie civil, le dimensionnement des fondations et de tout ouvrage en terre, tel que les chaussées, les couches d'assise, les remblais, les terre-pleins, etc., nécessite des campagnes de reconnaissance géotechnique permettant d'évaluer les caractéristiques physico-mécaniques des sols en place. Ces campagnes sont souvent réalisées à l'aide de pénétromètres dynamiques. Ces pénétromètres dynamiques sont utilisés principalement pour définir la stratigraphie des sols et pour évaluer la portance des sols. Dans certains, cas, les pénétromètres dynamiques sont aussi utilisés pour contrôler le compactage des ouvrages en terre. Plus généralement, les pénétromètres dynamiques permettent de collecter des données utiles à la caractérisation géomécanique des sols.

Un pénétromètre dynamique est un outil permettant d'évaluer la résistance que le sol oppose à l'enfoncement, par battage, d'un train de tiges pourvu, à son extrémité inférieure, d'une pointe. Le battage est réalisé à l'aide d'un marteau qui est levé puis lâché à plusieurs reprises depuis une hauteur constante. L'opérateur compte le nombre d'impacts nécessaires pour enfoncer la pointe dans le sol tous les « X » centimètres. Cette distance de « X » centimètres est soit tracée à l'aide d'une craie sur les tiges, soit marquée par usinage directement sur les tiges. Dans la mesure où les tiges présentent souvent une longueur fixe et les profondeurs d'investigation classiques en géotechnique sont bien supérieures à cette longueur fixe, l'opérateur doit rajouter systématiquement au train de tiges des tiges supplémentaires qui lui permettent d'incrémenter la profondeur d'enfoncement du train de tiges dans le sol. A la fin d'un sondage, le relevé de mesures effectué par l'opérateur permet d'évaluer la résistance opposée par le sol à la pénétration en fonction de la profondeur, par tranches de « X » centimètres.

De manière générale, un pénétromètre dynamique comprend un train de tiges pourvu, à son extrémité inférieure, d'une pointe typiquement conique, un marteau monté mobile sur un guide, et une enclume qui reçoit des impacts successifs appliqués par le marteau et qui les transmet au train de tiges en vue d'enfoncer la pointe dans le sol. Le marteau présente un poids fixe et se présente souvent sous la forme d'un cylindre plein, qui est monté coulissant le long d'une barre de guidage et qui peut comporter des poignées pour faciliter le levage du marteau pendant le sondage. Compte tenu de leur principe de conception et d'utilisation assez simple, il existe aujourd'hui une grande variété de pénétromètres dynamiques à travers le monde.

En Europe, les recommandations d'usage et les géométries pour les pénétromètres dynamiques sont fournies par la norme ISO 2246-2. Aux Etats-Unis, ainsi que dans de nombreux pays du continent américain, d'Asie et d'Afrique, les pénétromètres dynamiques répondent plutôt à la norme américaine ASTM-D6951-18 2015. Dans tous les cas, ces différents pénétromètres dynamiques se déclinent en différentes tailles et selon le niveau de l'énergie de battage, directement liée au poids du marteau : l'usage des pénétromètres dynamiques légers, pour lesquels le marteau pèse typiquement huit ou dix kilos, est très répandu en raison de leur faible encombrement et de leur facilité de transport, en permettant de caractériser un sol pour les cinq à sept premiers mètres de profondeur, tandis que les pénétromètres dynamiques lourds, pour lesquels le marteau peut peser cinquante kilos, nécessitent des moyens de transport et de mise en place plus importants.

Sur le terrain, le mode opératoire des sondages réalisés à l'aide d'un tel pénétromètre dynamique est le même, quel que soit le type de pénétromètre dynamique employé. Il s'agit d'enfoncer dans le sol la pointe du train de tiges, par battage à l'aide du marteau qui est levé à une hauteur fixe par rapport à l'enclume puis relâché par l'opérateur, et ce à différentes reprises. Pendant ces opérations, on compte le nombre d'impacts nécessaires pour enfoncer la pointe d'une distance de « X » centimètres dans le sol. Une fois que la pointe a été enfoncée de « X » centimètres dans le sol, on compte à nouveau le nombre de coups nécessaires pour enfoncer de « X » centimètres supplémentaires la pointe dans le sol. Ces opérations sont répétées à plusieurs reprises, jusqu'à la fin du sondage. Dans la pratique, lorsque le pénétromètre dynamique est dépourvu d'instrumentation de mesure associée, ces opérations sont réalisées par au moins deux opérateurs, à savoir un premier opérateur qui tient le pénétromètre avec une main et qui lève et laisse tomber le marteau depuis la hauteur fixe avec l'autre main, et un second opérateur qui compte et qui note le nombre d'impacts nécessaires pour faire pénétrer la pointe de « X » centimètres dans le sol, en prenant comme référence les marques sur le train de tiges. Le sondage est arrêté soit lorsque la profondeur souhaitée du sondage est atteinte, soit lorsqu'un refus est rencontré. De manière générale, le refus intervient lorsque l'énergie de battage, fournie par le marteau est insuffisante pour faire pénétrer la pointe dans le sol, par exemple en cas de frottement latéral trop important entre le train de tiges et le sol. Le sondage est également interrompu lorsque l'inclinaison du train de tiges par rapport à la verticale est trop élevée, typiquement supérieure à 10°. A la fin du sondage, le relevé des mesures effectué par le second opérateur permet, a posteriori, c'est-à-dire après que les mesures aient été traitées notamment avec un logiciel ad hoc, d'évaluer la résistance opposée par le sol à la pénétration en fonction de la profondeur et par tranches de « X » centimètres. La précision et la fiabilité des mesures relevées selon ce mode opératoire, qui est très répandu actuellement, dépendent de l'expertise des opérateurs, mais aussi de l'homogénéité du terrain.

Une difficulté ou limite liée à l'usage des pénétromètres dynamiques concerne l'énergie de battage fournie par l'impact du marteau au cours du sondage. Puisqu'il n'est pas possible de faire varier la hauteur de chute et le poids du marteau, on dit que l'énergie de battage reste constante. Dès lors, selon le pénétromètre utilisé, l'énergie de battage peut se révéler soit insuffisante pour traverser des sols ayant une consistance très dure, ce qui entraîne le refus et l'arrêt prématuré du sondage, soit trop importante pour l'évaluation de sols mous, ce qui entraîne des grands enfoncements et une dispersion importante dans les mesures. Etant donné que, dans la nature, les alternances de nature mais aussi de dureté des couches du sol peuvent être fréquentes, les opérateurs sont contraints soit d'arrêter le sondage et de déclarer le refus de ce dernier, soit d'accepter des inexactitudes des mesures.

Face à cette problématique, différentes propositions techniques ont été faites par le passé.

US 5 313 825 a ainsi proposé un pénétromètre dynamique dont le marteau comprend deux éléments distincts, qui sont montés coulissants sur un même guide et qui peuvent être solidarisés l'un à l'autre de manière réversible. L'opérateur utilise soit un seul des deux éléments pour appliquer des impacts, l'autre élément étant alors dégagé du pénétromètre, soit les deux éléments conjointement après les avoir fixés l'un à l'autre par des vis dédiées. Le marteau présente ainsi un poids qui peut être modifié selon qu'un seul ou que les deux éléments sont utilisés. Pour enlever ou rajouter le second élément vis-à-vis du premier élément du marteau, l'opérateur doit arrêter le sondage, dévisser les vis, typiquement à l'aide d'une clé spéciale, puis rajouter ou enlever le second élément. Le sondage peut ensuite reprendre. Ces opérations sont chronophages et pénibles. De plus, sur le terrain, le risque de perdre les vis et/ou la clé est important. Par ailleurs, le fait d'augmenter ou de diminuer le poids du marteau implique une précaution opératoire qui ne doit pas être oubliée par l'opérateur en charge du relevé des mesures : en effet, comme expliqué dans US 5 313 825, le relevé des mesures est manuel et l'opérateur doit donc obligatoirement indiquer, dans son suivi de sondage, quel(s) est ou sont le ou les éléments du marteau utilisé(s). A défaut, les interprétations ultérieures des mesures risquent d'être faussées, entraînant in fine des erreurs dans le calcul des fondations pour les ouvrages projetés sur le terrain.

Pour faciliter la mesure de l'enfoncement à l'issue de chaque impact, il est courant d'utiliser des règles graduées, placées verticalement de manière parallèle au train de tiges, ce qui permet de relever l'enfoncement obtenu impact après impact. Dans la plupart des cas, ces relevés sont effectués par un opérateur, ce qui nécessite du temps de lecture mais aussi un traitement ultérieur. Afin de s'affranchir de cette difficulté, des systèmes utilisant des règles magnétiques analogiques ont été également développés : ces systèmes permettent d'automatiser la mesure, mais ils sont encombrants et manquent de précision pour le cas des sols raides dans lesquels l'enfoncement est très faible à chaque impact. De plus, l'usage de ces règles demande un montage spécifique sur le pénétromètre dynamique, augmentant le volume de l'appareil et le temps de mise en œuvre.

De son côté, US 2007/0131025, qui peut être considéré comme l'état de la technique le plus proche de la présente invention, a proposé d'utiliser un boîtier de mesure, qui est équipé d'un capteur de distance émettant une onde et qui est placé par terre, à proximité immédiate du train de tiges. Une cible, telle qu'un plateau cylindrique, doit être fixée sur le pénétromètre, typiquement au niveau de l'enclume, afin de réfléchir l'onde envoyée par le capteur de distance. Le capteur de distance permet ainsi de mesurer l'enfoncement itératif du train de tiges, du fait du rapprochement progressif de la cible vis-à-vis du sol sur lequel repose le boîtier de mesure. Le boîtier de mesure est connecté à un dispositif de traitement séparé, tel qu'un ordinateur ou un téléphone intelligent, afin de connaître la distance mesurée par le capteur de distance et de suivre sa diminution itérative au fur et à mesure des impacts. Le boîtier embarque une batterie électrique, qui alimente le capteur de distance et une électronique pour la connexion avec le dispositif de traitement, l'autonomie de cette batterie étant toutefois limitée puisque le capteur de distance fonctionne en continu. Afin d'associer la diminution itérative de la distance mesurée par le capteur de distance à la succession des impacts appliqués par le marteau, un second capteur, indépendant du boîtier de mesure, doit être prévu sur le pénétromètre dynamique, ce second capteur étant sensible à la présence du marteau lorsque ce dernier est soulevé en haut du guide. Les mesures du second capteur sont également envoyées au dispositif de traitement afin que ce dernier les corrèle aux mesures provenant du capteur de distance. Le système de mesure de US 2007/0131025 s'avère ainsi complexe et peu adapté aux conditions difficiles sur le terrain, de par ses différents composants séparés et leur autonomie énergétique limitée. De plus, malgré le gain de temps dans le relevé des mesures, l'opérateur a besoin de réaliser de nombreuses opérations préalables pour l'implémentation de ce système de mesure à chaque fois qu'une tige supplémentaire est ajoutée au pénétromètre. En effet, à chaque ajout d'une nouvelle tige, l'opérateur doit, avant de poursuivre l'essai, démonter la cible depuis le train de tiges, raccorder la nouvelle tige puis repositionner et ajuster la cible contre le train de tiges.

Le but de la présente invention est de proposer un nouveau dispositif de mesure, qui soit plus pratique et plus performant.

A cet effet, l'invention a pour objet un dispositif de mesure pour la caractérisation géomécanique d'un sol, tel que défini à la revendication 1.

L'invention a également pour objet un système de mesure pour la caractérisation géomécanique d'un sol, tel que défini à la revendication 6.

Une des idées à la base de l'invention est d'agencer, dans un boîtier du dispositif de mesure, un système électronique regroupant un capteur de distance et un accéléromètre, ce boîtier étant conçu pour être rapporté et solidarisé, par tout moyen approprié, à un pénétromètre dynamique, notamment mais non exclusivement à l'enclume de ce pénétromètre dynamique. Le capteur de distance est prévu pour émettre une onde, typiquement optique ou sonore, en direction du sol contre lequel cette onde se réfléchit pour revenir au capteur de distance : en mesurant le temps nécessaire à l'onde pour effectuer le trajet aller-retour entre le capteur de distance et le sol, le capteur de distance permet de déterminer la distance entre lui et la surface du sol, autrement dit la distance entre le dispositif de mesure et la surface du sol. L'accéléromètre fournit, quant à lui, un signal de mesure représentatif de l'accélération du dispositif de mesure : à chaque impact appliqué à l'enclume et, à travers elle, au train de tiges, l'accélération du dispositif de mesure présente un pic d'amplitude. L'exploitation de ce signal de mesure permet avantageusement de détecter les différents impacts successifs et donc de les compter, tout en les associant à un enfoncement du train de tiges dans le sol, calculé à partir des distances que mesure le capteur de distance à l'issue de chaque impact. En pratique, les signaux de mesure provenant respectivement de l'accéléromètre et du capteur de distance sont traités par une unité de traitement du système électronique du dispositif de mesure, afin de disposer de données dont un traitement, en vue de détecter et ainsi dénombrer les impacts successifs et de déterminer l'enfoncement progressif correspondant du train de tiges, peut être opéré par cette unité de traitement et/ou par un dispositif de traitement ad hoc, qui est séparé du dispositif de mesure et du pénétromètre dynamique et qui, le cas échéant, est connecté sans fil à l'unité de traitement. Ce dispositif de traitement est par exemple un ordinateur, une tablette, un téléphone intelligent, une plateforme informatique, etc. Dans tous les cas, le dispositif de mesure selon l'invention fournit des données fiables et précises pour la caractérisation géomécanique du sol, tout en étant particulièrement pratique à utiliser, moyennant simplement la solidarisation de son boîtier au pénétromètre dynamique en veillant que le capteur de distance soit orienté vers le sol puisque ce dernier sert de référence fixe, et ce notamment sans utiliser d'autres composants à rapporter sur le pénétromètre dynamique.

Par ailleurs, comme détaillé par la suite, l'exploitation du signal de mesure provenant de l'accéléromètre permet avantageusement de renforcer la praticité du dispositif de mesure, en lien avec son autonomie énergétique ou encore avec l'utilisation d'un marteau de poids variable. Egalement comme détaillé par la suite, le dispositif de mesure selon l'invention présente avantageusement des aménagements structurels en lien avec son boîtier, ou encore des aménagements complémentaires du système électronique, renforçant ainsi les performances du dispositif de mesure.

Ainsi, des caractéristiques optionnelles avantageuses du dispositif et du système de mesure conformes à l'invention sont spécifiées aux revendications 2 à 5 et 7 à 11.

L'invention a également pour objet un procédé de mesure pour la caractérisation géomécanique d'un sol, tel que défini à la revendication 12.

Ce procédé de mesure peut être mis en oeuvre par le dispositif et le système de mesure, tels que définis plus haut.

Un aspect avantageux optionnel du procédé de mesure selon l'invention est spécifié à la revendication 13.

L'invention sera mieux comprise à la lecture de la description qui va suivre, donnée uniquement à titre d'exemple et faite en se référant aux dessins sur lesquels :
[Fig. 1] la figure 1 est une vue en perspective éclatée d'un système de mesure conforme à l'invention ;
[Fig. 2] la figure 2 est une coupe longitudinale d'un pénétromètre et d'un dispositif mesure, assemblés l'un à l'autre, du système de mesure de la figure 1 ;
[Fig. 3] la figure 3 est une vue à plus grande échelle du détail III sur la figure 2 ;
[Fig. 4] la figure 4 est une vue en perspective du dispositif de mesure montré sur les figures précédentes ;
[Fig. 5] la figure 5 est une vue en élévation selon la flèche V de la figure 4, montrant de manière schématique le contour extérieur du dispositif de mesure et l'intérieur d'un boîtier de ce dispositif de mesure ;
[Fig. 6] la figure 6 est une vue en élévation du système de mesure de la figure 1, en cours d'utilisation ; et
[Fig. 7] la figure 7 est une vue similaire à la figure 6, illustrant une étape d'utilisation du système de mesure, ultérieure à celle montrée sur la figure 6.

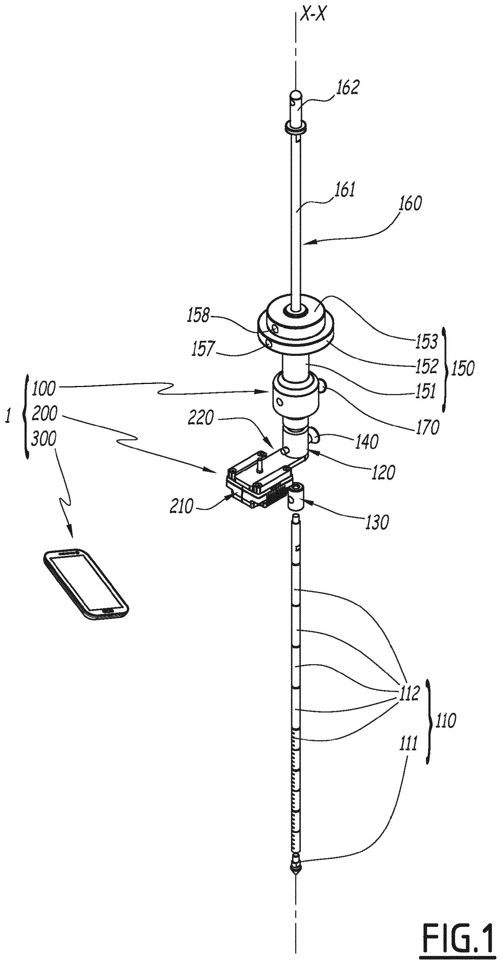

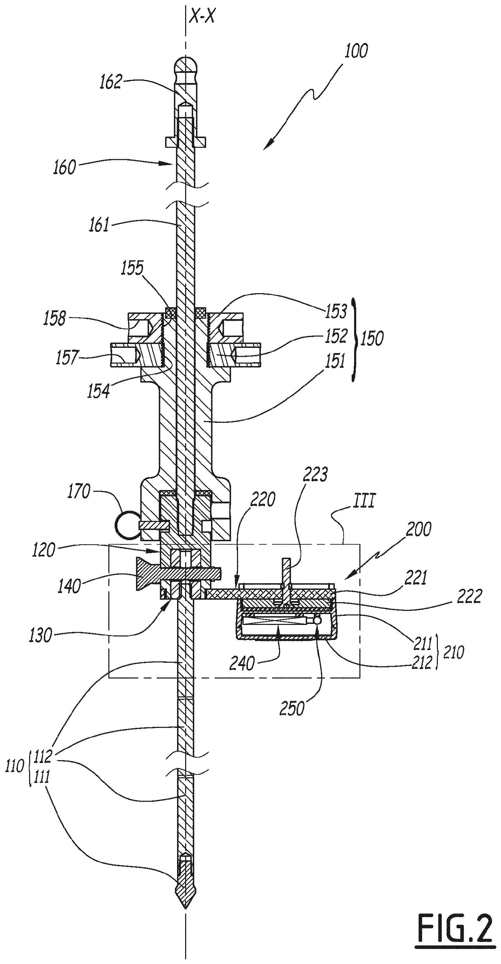

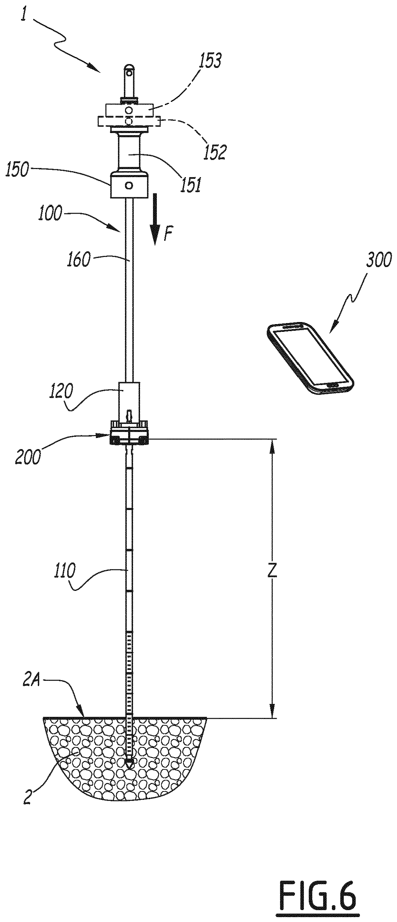

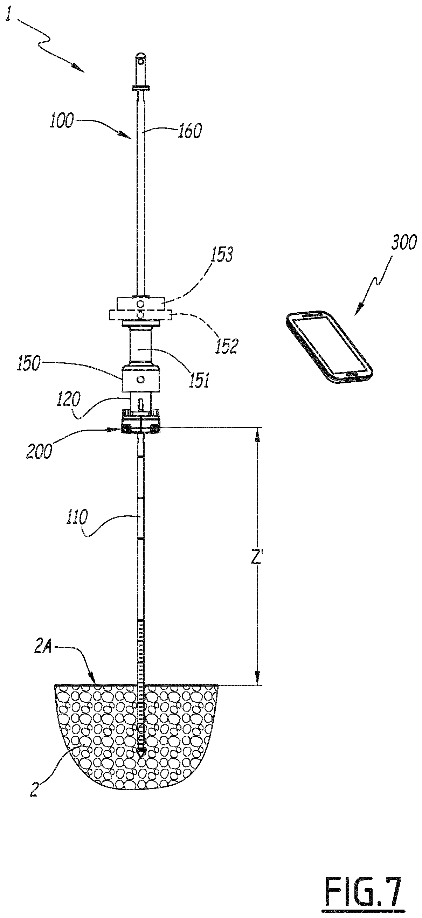

Sur les figures 1 à 7 est représenté un système de mesure 1 permettant de mesurer des caractéristiques géomécaniques d'un sol. Sur la figure 1, le système de mesure 1 est représenté dans une configuration non fonctionnelle, tandis que, sur les figures 6 et 7, le système de mesure 1 est représenté en cours d'utilisation sur un sol 2 d'un terrain dont on chercher à évaluer les caractéristiques physico-mécaniques. Sur les figures 2 à 5, seule une partie du système de mesure 1 est représentée.

Comme représenté sur les figures 1 à 3, 6 et 7, le système de mesure 1 comprend un pénétromètre dynamique 100. Le pénétromètre dynamique 100 définit un axe géométrique X-X, suivant lequel s'étend le pénétromètre dynamique 100 et qui, lorsque le système de mesure 1 est utilisé, est agencé à la verticale, c'est-à-dire rigoureusement à la verticale ou de manière faiblement inclinée par rapport à cette dernière, comme montré sur les figures 6 et 7.

Par commodité, la suite de la description est orientée par rapport à l'axe X-X en considérant que ce dernier s'étend à la verticale. Ainsi, les termes « bas », « inférieur » et « similaire » s'entendent comme étant orientés vers le sol 2, tandis que les termes « haut », « supérieur » et « similaire » s'entendent comme étant orientés à l'opposé du sol 2.

Comme bien visible sur les figures 1, 2, 6 et 7, le pénétromètre dynamique 100 comprend un train de tiges 110 qui est aligné et centré sur l'axe X-X. A son extrémité inférieure, le train de tiges 110 inclut une pointe 111 qui, comme dans l'exemple considéré ici, est conique, en étant centrée sur l'axe X-X et convergente vers le bas. De manière connue en soi, la section et l'angle d'ouverture de la pointe 111 sont spécifiques au pénétromètre dynamique 100, cet aspect n'étant pas limitatif de l'invention. Quelles que soient les spécificités de la pointe 111, cette dernière permet de déformer et cisailler le sol 2 lorsqu'un impact lui est appliqué vers le bas suivant l'axe X-X.

Le reste du train de tiges 110 comprend des tiges 112, qui se succèdent de manière alignée suivant l'axe X-X depuis la pointe 111, jusqu'à l'extrémité supérieure du train de tiges 110, formée par la partie terminale supérieure de la tige la plus haute parmi les tiges 112. Là encore, la dimension axiale et le diamètre des tiges 112 sont spécifiques au pénétromètre dynamique 100, cet aspect n'étant pas limitatif de l'invention. Dans tous les cas, les tiges 112 sont prévues pour se raccorder mécaniquement les unes aux autres, par exemple par vissage ou par emboîtement. Les tiges 112 permettent de transmettre à la pointe 111 un impact appliqué vers le bas suivant l'axe X-X à la tige la plus haute parmi les tiges 112, permettant ainsi au train de tiges 110 d'être enfoncé dans le sol 2.

Le pénétromètre dynamique 100 comprend également une enclume 120, qui est agencée à l'aplomb axial du train de tiges 110 et qui est conçue pour recevoir des impacts successifs vers le bas suivant l'axe X-X et les transmettre au train de tiges 110. La forme de réalisation de l'enclume 120 n'est pas limitative de l'invention du moment que, sur son côté inférieur, l'enclume est prévue pour être liée mécaniquement à l'extrémité supérieure du train de tiges 110 et transmettre à ce dernier un impact vers le bas suivant l'axe X-X. Dans l'exemple de réalisation considéré sur les figures, l'enclume 120 présente globalement une forme cylindrique, centrée sur l'axe X-X.

Le pénétromètre dynamique 100 comporte également un adaptateur 130 qui permet de joindre mécaniquement l'enclume 120 et le train de tiges 110. Comme bien visible sur les figures 1 à 3, l'adaptateur 130 est interposé, suivant l'axe X-X, entre l'enclume 120 et le train de tiges 110, en étant en appui axial à la fois contre l'enclume 120 et contre le train de tiges 110, plus précisément la tige la plus haute parmi les tiges 112 de ce dernier. Dans l'exemple de réalisation considéré sur les figures, l'adaptateur 130 présente une forme globalement cylindrique, centrée sur l'axe X-X, et est au moins partiellement reçu de manière complémentaire dans un logement 121 de l'enclume 120, débouchant sur le côté inférieur de l'enclume 120. En pratique, une goupille 140 est prévue pour retenir l'adaptateur 130 à l'intérieur du logement 121 et éviter ainsi un désassemblage inopiné, cette goupille 140 s'étendant en travers de l'enclume 120 et de l'adaptateur 130, notamment suivant une direction radiale à l'axe X-X. Sur son côté qui, sur les figures, est tourné vers le bas, l'adaptateur 130 présente à la fois une face 131, qui s'étend dans un plan géométrique perpendiculaire à l'axe X-X, et un taraudage 132, qui est centré sur l'axe X-X et qui débouche sur la face 131. Sur son côté opposé, c'est-à-dire sur son côté qui, sur les figures, est tourné vers le haut, l'adaptateur 130 présente à la fois une face 133, qui s'étend dans un plan géométrique perpendiculaire à l'axe X-X, et un alésage 134, qui est centré sur l'axe X-X et qui débouche sur la face 133.

Le taraudage 132 est prévu pour recevoir, par vissage, un filetage complémentaire que présente l'extrémité supérieure du train de tiges 110 considéré sur les figures : ainsi, à l'état assemblé du pénétromètre dynamique 100 considéré sur les figures, le filetage de l'extrémité supérieure du train de tiges 110 est vissé à fond dans le taraudage 132 jusqu'à mettre en appui axial un épaulement, prévu à la base de ce filetage, contre la face 131 de l'adaptateur 130, tandis que la face 133 de l'adaptateur 130 forme un appui axial vers le bas pour l'enclume 120, en particulier pour le fond du logement 121. Grâce à son alésage 134, l'adaptateur 130 peut être utilisé au sein du pénétromètre dynamique 100 dans une configuration différente de celle qui vient d'être décrite, lorsque l'extrémité supérieure du train de tiges 110 est dépourvue d'un filetage mais est réalisée sous forme d'un téton cylindrique lisse, non envisagé sur les figures : lorsque l'extrémité supérieure du train de tiges 110 est formée d'un tel téton cylindrique lisse, l'adaptateur 130 est utilisé dans une position retournée par rapport à celle illustrée sur les figures, c'est-à-dire de manière que sa face 133 soit tournée vers le bas et que l'alésage 134 reçoive le téton cylindrique lisse jusqu'à mettre en appui axial un épaulement, prévu à la base de ce téton, contre la face 133, tandis que, dans le même temps, la face 131 de l'adaptateur 130 forme un appui axial vers le bas pour l'enclume 120, en particulier pour le fond du logement 121. L'adaptateur 130 permet ainsi d'utiliser l'enclume 120 avec deux types différents de trains de tiges, à savoir le train de tiges 110 montré sur les figures, ayant son extrémité supérieure qui est filetée, et un autre train de tiges, dont l'extrémité supérieure est cylindrique lisse.

Le pénétromètre dynamique 100 comporte par ailleurs un marteau 150 qui est monté mobile sur un guide 160 solidaire de l'enclume 120. Dans l'exemple de réalisation considéré sur les figures, le marteau 150 présente une forme globale tubulaire, qui est centrée sur l'axe X-X et dont l'alésage central reçoit une barre 161 du guide 160, s'étendant vers le haut depuis l'enclume 120 en étant liée solidairement à cette dernière par tout moyen approprié. Le marteau 150 est mobile suivant l'axe X-X le long de la barre 161, et ce entre une position haute, qui est illustrée sur la figure 6 et dans laquelle le marteau est écarté de l'enclume 120, et une position basse, qui est illustrée sur la figure 7 et dans laquelle le marteau 150 est au contact de l'enclume 120, de manière que le marteau 150 applique à l'enclume 120 un impact vers le bas suivant l'axe X-X lorsqu'il passe de la position haute à la position basse. En pratique, lors de l'utilisation du pénétromètre dynamique 100, le marteau 150 est soulevé jusqu'à la position haute, avant d'être relâché pour que, sous l'effet de la gravité, il tombe sur l'enclume 120. La position haute est repérée sur le guide 160 afin que l'écart entre l'enclume 120 et le marteau 150 en position haute soit prédéterminé à une valeur qui reste fixe à chaque fois que le marteau est soulevé jusqu'à cette position haute : dans l'exemple de réalisation considéré sur les figures, ce repérage de la position haute est réalisé à l'aide d'une butée prévue à l'extrémité supérieure du guide 160, contre laquelle le marteau 150 en position haute est amené en contact. Cette butée est avantageusement intégrée à une poignée 162 coiffant la barre 161.

Comme bien visible sur les figures 1 et 2, le marteau 150 inclut avantageusement plusieurs éléments de marteau distincts dont au moins un est amovible par rapport au reste du marteau 150 et, par-là, par rapport au reste du pénétromètre dynamique 100.

Plus précisément, dans l'exemple de réalisation considéré sur les figures, le marteau 150 inclut ainsi un premier élément de marteau 151 qui, dans le marteau, coopère par complémentarité de formes avec le guide 160 lors du déplacement du marteau entre les positions haute et basse, notamment en coulissant autour et le long de la barre 161. Le premier élément de marteau 151 présente une forme tubulaire, centrée sur l'axe X-X.

L'extrémité inférieure de ce premier élément de marteau 151 est prévue pour se retrouver au contact de l'enclume 120 lorsque le marteau 150 est dans la position basse, de manière à pouvoir appliquer à cette dernière un impact vers le bas suivant l'axe X-X. L'extrémité inférieure du premier élément de marteau 151 présente avantageusement une forme en cloche de manière à recevoir intérieurement au moins une partie de l'enclume 120 lorsque le marteau 150 est en position basse : cette forme en cloche permet d'atténuer l'intensité du bruit généré lors de l'impact du marteau 150 sur l'enclume 120, par effet de confinement de ce bruit à l'intérieur de la cloche. Suivant un autre aspect optionnel avantageux qui améliore l'ergonomie du marteau 150, la partie courante du premier élément de marteau 151, c'est-à-dire la partie de ce dernier, qui relie l'une à l'autre les extrémités inférieure et supérieure du premier élément de marteau 151, est façonnée pour faciliter la prise en main du premier élément de marteau 151 et, par-là, du marteau 150. Dans l'exemple de réalisation considéré sur les figures, cette partie courante du premier élément de marteau 151 présente ainsi une forme cylindrique à section circulaire dont le diamètre est inférieur aux dimensions transversales respectives des extrémités inférieure et supérieure du premier élément de marteau 151. Par ailleurs, une goupille 170, agencée en travers de l'extrémité inférieure du premier élément de marteau 151 et en travers de la partie de l'enclume 120, reçue dans cette extrémité inférieure du premier élément de marteau 151, peut être prévue pour retenir assemblée l'enclume 120 et le premier élément de marteau 151 lorsque le marteau 150 n'est pas utilisé aux fins d'appliquer un impact à l'enclume, par exemple lors du transport et de la mise en place du pénétromètre dynamique 100.

Le marteau 150 inclut également un deuxième élément de marteau 152 et un troisième élément de marteau 153 qui sont conçus pour être chacun rapportés de manière amovible sur le premier élément de marteau 151. A cet effet, chacun des éléments de marteau 152 et 153 est pourvu d'un taraudage 154, 155, qui est centré sur l'axe X-X et qui est prévu pour être vissé sur un filetage complémentaire 156 du premier élément de marteau 151. Dans l'exemple considéré sur les figures, le filetage 156 est prévu à l'extrémité supérieure du premier élément de marteau 151. Dans tous les cas, chacun des éléments de marteau 152 et 153 est ainsi vissable directement sur le premier élément de marteau 151, et ce, d'une part, sans avoir à désassembler le premier élément de marteau 151 vis-à-vis du guide 160 et, d'autre part, sans nécessiter de pièces rapportées supplémentaires, telles que des vis. Avantageusement, pour faciliter l'entraînement en rotation de chacun des éléments de marteau 152 et 153 aux fins de leur vissage-dévissage vis-à-vis du filetage 156, chacun des éléments de marteau 152 et 153 est pourvu d'au moins un trou latéral 157, 158, qui s'étend suivant une direction radiale à l'axe X-X et qui est adapté pour recevoir l'une des deux extrémités d'une tige similaire aux tiges 112 : de cette façon, sur le terrain, un opérateur peut utiliser une des tiges 112, non encore utilisée au sein du train de tiges 110, pour en introduire une extrémité dans le trou 157, 158 et ainsi appliquer plus facilement un couple de vissage-dévissage à l'élément de marteau correspondant 152, 153 aux fins de l'assemblage ou du désassemblage de cet élément de marteau 152, 153 vis-à-vis du premier élément de marteau 151.

Quelles que soient les spécificités des éléments de marteau 151, 152 et 153 du marteau 150, on comprend que le poids du marteau 150 change selon la présence effective de l'un et/ou de l'autre des éléments de marteau 152 et 153, en plus de la présence systématique du premier élément de marteau 151 dans le marteau 150.

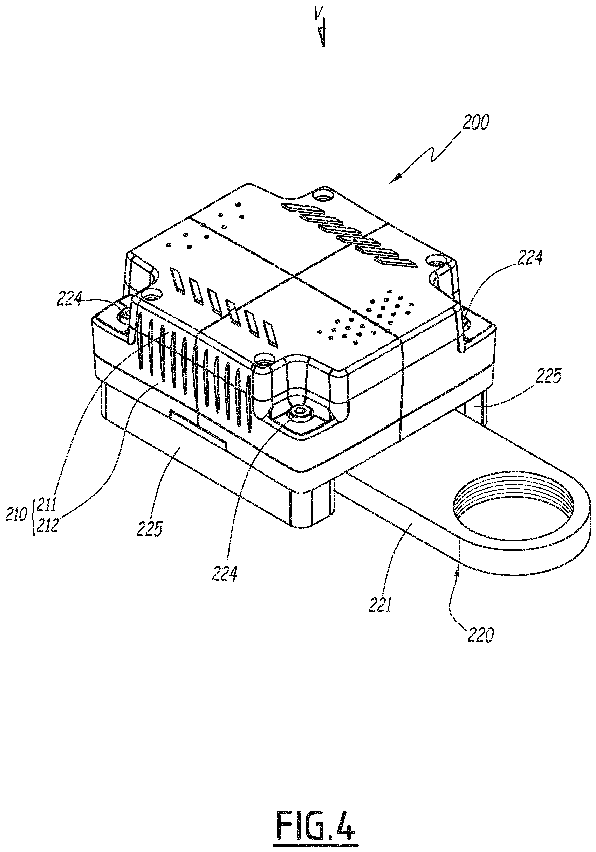

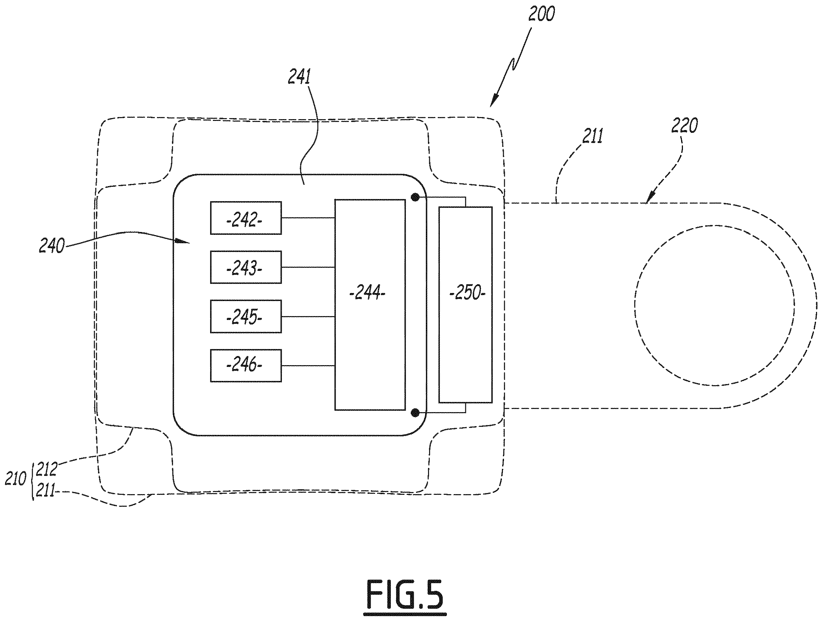

Comme bien visible sur les figures 1 à 3, le système de mesure 1 comprend, en plus du pénétromètre dynamique 100 décrit jusqu'ici, un dispositif de mesure 200, qui est représenté seul sur les figures 4 et 5.

Le dispositif de mesure 200 comporte un boîtier 210 qui, dans l'exemple de réalisation considéré sur les figures, inclut une coque principale 211 et un couvercle 212. En pratique, le boîtier 210 est avantageusement réalisé en une matière plastique, tout en étant prévu pour résister aux chocs et, plus généralement, aux conditions d'utilisation en extérieur, sur un terrain à évaluer.

Le boîtier 210 est adapté pour être solidarisé, en particulier de manière amovible, au pénétromètre dynamique 100. A cet effet, dans l'exemple de réalisation considéré sur les figures, le dispositif de mesure 200 comprend des moyens de solidarisation mécanique 220 dont la forme de réalisation, envisagée dans l'exemple des figures, est détaillée juste ci-après.

Les moyens de solidarisation mécanique 220 comprennent une plaque 221, notamment métallique, qui s'étend de manière transversale à l'axe X-X. A son extrémité tournée vers l'axe X-X, la plaque 221 est solidarisée mécaniquement au pénétromètre dynamique, en coopérant directement avec ce dernier. Dans l'exemple considéré sur les figures, la plaque 221 est ainsi pourvue d'un taraudage qui, aux fins de la solidarisation au pénétromètre dynamique 100, est vissée autour d'un filetage complémentaire de l'enclume 120.

A son extrémité opposée à l'axe X-X, la plaque 221 est solidarisée mécaniquement au boîtier 210. A cet effet, dans l'exemple de réalisation considéré sur les figures, les moyens de solidarisation mécanique 220 comprennent une semelle 222, notamment métallique, qui, d'une part, est reliée mécaniquement à la plaque 221 par un boulon 223 et, d'autre part, est reliée à la coque principale 211 du boîtier 10 par des vis 224. Pour renforcer l'assemblage fixe entre la plaque 221 et la semelle 222, les moyens de solidarisation mécanique 220 comprennent également des cales 225, qui sont agencées le long et de part et d'autre de la plaque 221 et qui sont reliées à la semelle 222 par les vis 224.

La forme de réalisation des moyens de solidarisation mécanique 220, qui vient d'être décrite n'est pas limitative de l'invention, dans le sens où de multiples formes de réalisation peuvent être envisagées pour les moyens de solidarisation mécanique 220 du moment que ces derniers solidarisent le boîtier 210 au pénétromètre dynamique 100, notamment à son enclume 120, par une liaison mécanique réversible.

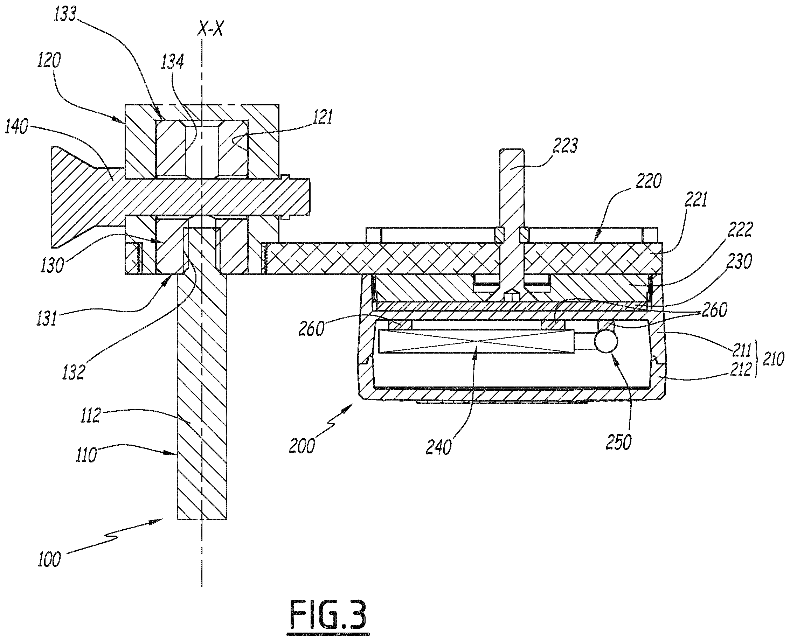

Quelle que soit la forme de réalisation des moyens de solidarisation mécanique 220, le dispositif de mesure 200 comprend avantageusement au moins un amortisseur 230, typiquement en caoutchouc ou en un matériau élastomère similaire, qui est interposé entre le boîtier 210 et les moyens de solidarisation mécanique 220 de manière à limiter la transmission au boîtier 210 de vibrations provenant du pénétromètre dynamique 100. Dans l'exemple de réalisation considéré sur les figures, l'amortisseur 230 est interposé entre le boîtier 210 et la semelle 222, comme illustré schématiquement sur la figure 3.

Comme plus spécifiquement montré sur les figures 3 et 5, le dispositif de mesure 200 comprend également un système électronique 240.

Ce système électronique 240 est logé à l'intérieur du boîtier 210, en étant notamment reçu dans un volume interne de ce boîtier, délimité par la coque principale 211 et le couvercle 212. En pratique, ce volume interne du boîtier 210 est étanché vis-à-vis de l'extérieur du boîtier 210 par tout moyen approprié intégré au boîtier 210, de manière à préserver de l'eau et de l'humidité le système électronique 240.

Comme représenté schématiquement sur la figure 5, le système électronique 240 comporte plusieurs composants ou groupes de composants, qui vont être détaillés ci-après et qui sont avantageusement portés sur une carte électronique 241 commune.

Le système électronique 240 comprend ainsi un accéléromètre 242 à même de mesurer l'accélération du système électronique 240 et, par-là, du dispositif de mesure 200. Les spécificités de l'accéléromètre 242 ne sont pas limitatives de l'invention.

Le système électronique 240 comprend également un capteur de distance 243 à même de déterminer la distance le séparant d'une surface vers laquelle ce capteur de distance 243 est orienté. A cet effet, le capteur de distance 243 est conçu pour mesurer le temps nécessaire à une onde, qu'il émet, pour effectuer un trajet aller-retour entre lui et la surface vers laquelle il est orienté. En pratique, l'onde précitée est une onde lumineuse, par exemple dans le domaine de l'infrarouge, ou une onde sonore. Dans tous les cas, le capteur de distance 243 intègre, à la fois, un émetteur à même d'émettre cette onde et un récepteur à même de détecter l'onde revenant au capteur de distance 243 après avoir été réfléchie, au moins partiellement, par la surface vers laquelle le capteur de distance 243 est orienté. Ainsi, le capteur de distance 243 est couramment appelé capteur de distance « TOF » (« TOF » étant l'acronyme de l'expression anglaise « Time Of Flight »). Lorsque le boîtier 210 est solidarisé au pénétromètre 100 et que le système de mesure 1 est en cours d'utilisation, le capteur de distance 243 est orienté vers le sol 2 à évaluer, de manière que le capteur de distance 243 permet de déterminer la distance entre le dispositif de mesure 200 et la surface 2A du sol 2. On comprend que l'agencement du capteur de distance 243 à l'intérieur du boîtier 210 et l'agencement de solidarisation du dispositif de mesure 200 sur le pénétromètre dynamique 100 sont prévus en conséquence, c'est-à-dire sont prévus pour que le capteur de distance 243 soit orienté vers le sol 2 lors de l'utilisation du système de mesure 1. Dans l'exemple de réalisation considéré sur les figures, le capteur de distance 243 est orienté vers le couvercle 212 du boîtier 210 et, lorsque le système de mesure 1 est utilisé comme sur les figures 6 et 7, le couvercle 212 est orienté vers la surface 2A du sol 2. Bien entendu, le couvercle 212 est réalisé en un matière qui laisse passer l'onde émise par le capteur de distance 243.

Le système électronique 240 comprend aussi une unité de traitement 244 qui, comme indiqué schématiquement sur la figure 5, est reliée à l'accéléromètre 242 et au capteur de distance 243. L'unité de traitement 244 est ainsi prévue pour recevoir les signaux de mesure provenant respectivement de l'accéléromètre 242 et du capteur de distance 243. L'unité de traitement 244 est adaptée pour appliquer un premier traitement à ces signaux de mesure, étant noté que les spécificités relatives à ce premier traitement seront données plus loin. En pratique, l'unité de traitement 244 comprend à la fois des composants électroniques analogiques et des composants électroniques numériques, afin de mettre en oeuvre le premier traitement précité.

Suivant des dispositions optionnelles dont l'intérêt apparaîtra plus loin, le système électronique 240 comprend également :
- une interface de communication sans fil 245 à même d'émettre vers l'extérieur du dispositif de mesure 200, selon un protocole de communication sans fil, les données résultant du premier traitement appliqué par l'unité de traitement 244, le protocole précité étant avantageusement à basse consommation d'énergie ; cette interface de communication sans fil 245 est, par exemple, une interface Wifi ou bien une interface Bluetooth, notamment à basse consommation couramment appelée interface « BLE » (acronyme de l'expression anglaise « « Bluetooth Low Energy ») ;
- un capteur d'inclinaison 246 à même de mesurer l'inclinaison du dispositif de mesure 200 par rapport à la verticale ; le capteur d'inclinaison 246 est par exemple un gyroscope électronique ; et
- un ou plusieurs autres capteurs sensibles aux conditions d'utilisation du dispositif de mesure 200, tels qu'un capteur de température, un capteur de luminosité, un capteur de localisation, etc.

Bien entendu, l'interface de communication sans fil 245, le capteur d'inclinaison 246 et les éventuels autres capteurs sont reliés à l'unité de traitement 244, comme indiqué schématiquement sur la figure 5, aux fins du traitement de leurs signaux respectifs par cette unité de traitement.

Le dispositif de mesure 200 comprend par ailleurs une batterie d'alimentation électrique 250, qui est logée à l'intérieur du boîtier 210 et qui, comme indiqué schématiquement sur la figure 5, est reliée au système électronique 240 afin d'alimenter en électricité les différents composants de ce système électronique 240. La batterie d'alimentation électrique 250 est par exemple branchée sur la carte électronique 241. La forme de réalisation de cette batterie d'alimentation électrique 250 n'est pas limitative de l'invention.

Comme indiqué schématiquement sur la figure 3, le dispositif de mesure 200 comprend aussi des amortisseurs 260, qui sont interposés entre le boîtier 210 et le système électronique 240, notamment la carte électronique 241 de ce dernier, ainsi que, le cas échéant, entre le boîtier 210 et la batterie d'alimentation électrique 250. Ces amortisseurs 260 permettent de limiter l'exposition du système électronique 240 et de la batterie d'alimentation électrique 250 aux vibrations provenant du pénétromètre dynamique 100 lors de l'utilisation du système de mesure 1. La durée de vue du système électronique 240 et de la batterie d'alimentation électrique 250 n'est ainsi pas altérée, malgré la solidarisation du boîtier 210 au pénétromètre dynamique 100 alors que ce dernier est le lieu d'impacts répétés lors de l'utilisation du système de mesure 1.

Enfin, le système de mesure 1 comprend un dispositif de traitement 300, comme illustré par les figures 1, 6 et 7. Ce dispositif de traitement 300 est distinct du pénétromètre dynamique 100 et du dispositif de mesure 200, en étant physiquement séparé du pénétromètre dynamique 100 et du dispositif de mesure 200.

Le dispositif de traitement 300 est adapté pour appliquer un second traitement aux données qui résultent du premier traitement appliqué par l'unité de traitement 244 aux signaux de mesure provenant de l'accéléromètre 242 et du capteur de distance 243, ainsi que, le cas échéant, provenant du capteur d'inclinaison 246 et des éventuels autres capteurs du système électronique 240. Les spécificités relatives à ce second traitement seront fournies un peu plus loin.

Dans tous les cas, le dispositif de traitement 300 est prévu pour recevoir les données résultant du premier traitement appliqué par l'unité de traitement 244. Dans l'exemple de réalisation considéré sur les figures, le dispositif de traitement 300 inclut à cet effet une interface de communication sans fil, qui est compatible avec l'interface de communication sans fil 245 du système électronique 240. Plus généralement, le système électronique 240 du dispositif de mesure 200 et le dispositif de traitement 300 sont prévus pour échanger des données, et ce sans fil via un protocole de communication ad hoc comme envisagé sur les figures, ou bien par l'intermédiaire d'une liaison filaire, par exemple USB, ou d'un support mémoire amovible, par exemple une carte mémoire.

Le dispositif de traitement 300 comprend des composants électroniques numériques permettant de mettre en oeuvre le second traitement précité. La forme de réalisation de ces composants électroniques numériques n'est pas limitative de l'invention. A titre d'exemple, le dispositif de traitement 300 comporte, voire est constitué d'un terminal mobile, tel qu'un ordinateur portable, une tablette ou un téléphone mobile intelligent, ou bien une plateforme informatique.

On va maintenant décrire un procédé de mesure, qui est mis en œuvre avec le système de mesure 1 et au travers duquel les premier et second traitements respectivement appliqués par l'unité de traitement 244 du dispositif de mesure 200 et par le dispositif de traitement 300 vont être présentés plus en détail. Ce procédé de mesure est décrit plus spécifiquement en lien avec les figures 6 et 7 sur lesquelles le système de mesure 1 est en cours d'utilisation sur le sol 2.

Sur la figure 6, le train de tiges 110 du pénétromètre 100 est déjà partiellement enfoncé dans le sol 2, la pointe 111 étant située au-dessous de la surface 2A du sol 2. Cet enfoncement partiel résulte d'impacts qui ont été appliqués à l'enclume 120 par le marteau 150 antérieurement à l'étape d'utilisation illustrée à la figure 6. Comme déjà évoqué plus haut, on rappelle que, pour appliquer un impact à l'enclume 120, le marteau 150 est écarté de l'enclume 120 en étant soulevé jusqu'à la position haute, avantageusement repérée par la poignée 162, puis le marteau 150 est relâché pour qu'il tombe sur l'enclume 120, en passant ainsi dans la position basse.

A l'issue du dernier impact avant l'étape illustrée à la figure 6, l'intervalle qui sépare le capteur de distance 243 et la surface 2A du sol 2 est déterminé en utilisant le capteur de distance 243 qui, à cet effet, mesure le temps nécessaire à l'onde qu'il émet pour effectuer un trajet aller-retour entre lui et la surface 2A du sol 2. L'intervalle précité correspond à une distance, notée Z sur la figure 6, entre le dispositif de mesure 200 et la surface 2A du sol 2.

A l'étape illustrée à la figure 6, le marteau 150 est dans la position haute et est relâché afin de passer vers la position basse, comme indiqué par la flèche F. Lorsque le marteau 150 atteint effectivement la partie basse, il applique un impact sur l'enclume 120 qui le transmet au train de tiges 110, ce train de tiges 110 s'enfonçant alors davantage dans le sol 2 : le système de mesure 1 se retrouve alors dans l'état illustré à la figure 7.

Lors de l'impact appliqué à l'enclume 120 pour passer de la figure 6 à la figure 7, l'enclume 120 et, par conséquent, le dispositif de mesure 200 solidarisé à cette dernière subissent une accélération substantielle, c'est-à-dire dont l'amplitude est forte, typiquement de plusieurs g. Cette accélération est mesurée en utilisant l'accéléromètre 242. L'impact correspondant est détecté à partir de l'accélération ainsi mesurée, en particulier à partir d'un pic d'amplitude de cette accélération mesurée.

A l'issue de l'impact appliqué pour passer de la figure 6 à la figure 7, la distance, notée Z' sur la figure 7, entre le dispositif de mesure 200 et la surface 2A du sol 2 est mesurée en utilisant le capteur de distance 243, de la même façon qu'avait été antérieurement mesurée la distance Z. L'enfoncement du train de tiges 110 dans le sol 2 entre les états respectivement illustrés par les figures 6 et 7 est alors déterminé à partir des distances mesurées Z et Z', cet enfoncement correspondant à la différence entre la distance Z et la distance Z'.

On comprend qu'en réitérant successivement des impacts sur l'enclume 120 en utilisant le marteau 150, ces différents impacts successifs sont individuellement détectés à partir des accélérations mesurées par l'accéléromètre 242. Le nombre de ces impacts peut ainsi être compté. Dans un même temps, l'enfoncement progressif du train de tiges 110 est déterminé à l'issue de chacun de ces impacts, à partir des distances mesurées par le capteur de distance 243.

Des données numériques représentatives du dénombrement des différents impacts successivement appliqués et de l'enfoncement du train de tiges 110 à l'issue de chaque impact permettent de calculer des caractéristiques géomécaniques du sol 2, en particulier des valeurs de résistance du sol 2. Par exemple, un indice de pénétration pour le sol 2 peut ainsi être calculé, notamment à partir de la formule de battage des Hollandais ou d'autres formules connues dans la littérature.

En pratique, les données numériques mentionnées juste ci-dessus sont fournies soit exclusivement par l'unité de traitement 244, via le premier traitement qu'elle applique aux signaux de mesure provenant de l'accéléromètre 242 et du capteur de distance 243, soit conjointement par l'unité de traitement 244 et le dispositif de traitement 300, via successivement le premier traitement, qu'applique l'unité de traitement 244 aux signaux de mesure provenant de l'accéléromètre 242 et du capteur de distance 243, et le second traitement, qu'applique le dispositif de traitement 300 aux données résultant du premier traitement.

En d'autres termes, selon une première possibilité de réalisation du système de mesure 1, l'unité de traitement 244 est adaptée, via le premier traitement, pour détecter les différents impacts successifs à partir des signaux de mesure provenant de l'accéléromètre 242 et pour déterminer l'enfoncement du train de tiges 110 dans le sol 2 à l'issue de chaque étape à partir des signaux de mesure provenant du capteur de distance 243. Dans ce cas, le dispositif de traitement 300 n'intervient pas pour fournir les données numériques représentatives du dénombrement des impacts et de l'enfoncement progressif du train de tiges 110, mais ce dispositif de traitement 300 est, le cas échéant, utilisé pour consulter ces données et/ou les mémoriser et/ou les transmettre vers des serveurs distants, notamment à des fins de post-traitement plus sophistiqués que le premier traitement appliqué par l'unité de traitement 244.

Selon une autre possibilité de réalisation du système de mesure 1, l'unité de traitement 244 et le dispositif de traitement 300 sont conjointement adaptés, via les premier et second traitements, pour détecter les différents impacts successifs à partir des signaux de mesure provenant de l'accéléromètre 242 et pour déterminer l'enfoncement du train de tiges 110 dans le sol 2 à l'issue de chaque impact à partir des signaux de mesure provenant du capteur de distance 243. Dans ce cas, le premier traitement, mis en œuvre par l'unité de traitement 244, peut simplement être conversion analogique-numérique, en convertissant les signaux de mesure en des données exploitables pour le second traitement, mis en œuvre par le dispositif de traitement 300. Plus généralement, on comprend que l'exploitation des signaux de mesure provenant de l'accéléromètre 242 et du capteur de distance 243 peut être limitée au niveau de l'unité de traitement 244, via le premier traitement, sous réserve que le dispositif de traitement 300 soit prévu pour mener à son terme cette exploitation, via le second traitement : dans ce cas, l'unité de traitement 244 peut être réduite à une forme de réalisation minimale. A l'inverse, si les capacités de traitement du dispositif de traitement 300 sont limitées ou incertaines, une partie substantielle de cette exploitation peut être prévue par l'unité de traitement 244, via le premier traitement.

Ainsi, dans tous les cas, les signaux de mesure provenant de l'accéléromètre 242 et les signaux de mesure provenant du capteur de distance 243 sont traités au moins par l'unité de traitement 244.

Bien entendu, ce que vient d'être décrit en détail pour les signaux de mesure provenant de l'accéléromètre 242 et du capteur de distance 243 s'applique aux signaux de mesure provenant du capteur d'inclinaison 246 et des éventuels autres capteurs du système électronique 240. En particulier, les données obtenues à partir des signaux de mesure provenant du capteur d'inclinaison 246 peuvent ensuite être stockées à des fins de contrôle.

Suivant un aspect optionnel du procédé de mesure, l'accélération mesurée à chaque impact est avantageusement utilisée pour déterminer quel(s) élément(s) de marteau du marteau 150, parmi les éléments de marteau 151, 152 et 153, est ou sont effectivement présent(s) dans le marteau ayant été utilisé pour appliquer l'impact. En effet, on notera que lors des étapes d'utilisation illustrées par les figures 6 et 7, ainsi que lors de chacune des étapes d'utilisation antérieures et postérieures, le marteau 150 est utilisable avec seulement l'élément de marteau 151 ou avec l'élément de marteau 151 et l'un et/ou l'autre des éléments de marteau 152 et 153, comme indiqué schématiquement en traits pointillés et en traits mixtes sur les figures 6 et 7. A cet effet, soit exclusivement le premier traitement, soit successivement les premier et second traitements sont prévus pour exploiter les signaux de mesure provenant de l'accéléromètre 242 afin de déterminer si, en plus du premier élément de marteau 151 systématiquement présent dans le marteau 50, l'un et/ou l'autre des éléments de marteau 152 et 153 sont présents dans le marteau 150. Ce calcul est mené à l'aide d'une fonction de transfert qui associe, moyennant un étalonnage préalable du système de mesure 1, la masse effective du marteau 150 et l'accélération mesurée par l'accéléromètre 242. En d'autres termes, lors de chaque impact, l'unité de traitement 244 ou bien l'unité de traitement 244 et le dispositif de traitement 300 sont à même de déceler celui ou ceux des éléments de marteau, parmi les éléments de marteau 151, 152 et 153, qui sont effectivement présents dans le marteau 150. En ajoutant ou en enlevant les éléments de marteau 152 et 153 au marteau 150, l'utilisateur peut donc facilement changer le poids du marteau 150 en cours de sondage, notamment pour s'adapter aux couches de sol rencontrées lors du sondage, sans avoir à se soucier de consigner cette information puisque le système de mesure 1 détermine, par lui-même, cette information à partir des signaux de mesure provenant de l'accéléromètre 242, étant souligné que cette information est déterminante pour calculer certaines caractéristiques géomécaniques du sol.

Selon un autre aspect optionnel relatif à l'exploitation des signaux de mesure provenant de l'accéléromètre 242, l'autonomie énergétique du dispositif de mesure 200 peut être substantiellement améliorée. A cet effet, le système électronique 240 est avantageusement prévu pour passer entre un état de fonctionnement, dans lequel la batterie d'alimentation électrique 250 alimente tout le système électronique 240, permettant ainsi la mise en œuvre du procédé décrit jusqu'ici, et un état de veille, dans lequel l'unité de traitement 244 interrompt l'alimentation, par la batterie d'alimentation électronique 250, du capteur de distance 243, ainsi que, avantageusement, de tous les autres composants du système électronique 240 hormis l'accéléromètre 242 et l'unité de traitement elle-même. L'unité de traitement 244 est alors adaptée pour, d'une part, passer le système électronique de l'état de veille à l'état de fonctionnement lorsque l'amplitude de l'accélération mesurée par l'accéléromètre 242 dépasse un seuil prédéterminé, valant par exemple 1 g environ, et, d'autre part, pour passer de l'état de fonctionnement à l'état de veille lorsque l'amplitude de l'accélération mesurée par l'accéléromètre 242 reste inférieure au seuil précité pendant une durée prédéterminée, valant par exemple 5 minutes. Il en résulte que, en dehors d'un sondage, le système électronique 240 est en veille et ne consomme donc que très peu d'électricité, préservant la batterie d'alimentation électrique 250. Lors d'un sondage, l'opérateur « réveille » le système électronique 240, c'est-à-dire le fait passer de l'état de veille à l'état de fonctionnement, en lui faisant subir une accélération suffisamment forte, qui, en pratique, correspond à un choc qu'applique l'opérateur soit directement sur le dispositif de mesure 200 à la main, soit sur le pénétromètre 200, notamment à l'aide du marteau 150. Pendant le déroulement du sondage, les impacts successifs du marteau 150 sur l'enclume 120 « maintiennent éveillée » le système électronique 240, c'est-à-dire font que le système électronique 240 reste dans l'état de fonctionnement. Une fois le sondage terminé, le système électronique 240 reste encore dans l'état de fonctionnement pendant la durée prédéterminée précitée, à l'issue de laquelle l'unité de traitement 244 commande le passage en état de veille du système électronique 240. Ainsi, pendant le stockage et le transport du dispositif de mesure 200, ainsi que pendant l'assemblage du dispositif de mesure 200 sur le pénétromètre dynamique 100, le système électronique 240 reste en état de veille, étant entendu que les accélérations que subit le dispositif de mesure 200 pendant ces opérations de stockage, de transport et d'assemblage sont insuffisantes en amplitude pour passer le système électronique 240 dans l'état de fonctionnement.

Par ailleurs, divers aménagements et variantes au système de mesure 1 décrit jusqu'ici, ainsi qu'au procédé de mesure présenté ci-dessus, sont envisagés ci-dessous, en pouvant être considérés isolément ou combinables entre eux :
- En complément ou en remplacement des moyens de solidarisation mécanique 220, le dispositif de mesure 200 peut comporter des moyens de solidarisation magnétique permettant de solidariser le boîtier 210 au pénétromètre dynamique 100 par une liaison aimantée. Par exemple, le boîtier 210 est alors équipé d'un ou de plusieurs aimants permanents qui, par effet magnétique avec une partie en acier du pénétromètre dynamique 100, se fixent fermement à ce dernier.
- Comme évoqué plus haut, la région du pénétromètre dynamique 100 sur laquelle le dispositif de mesure 200 est rapporté de manière fixe n'est pas limitée à l'enclume 120. En effet, moyennant l'adaptation des moyens de solidarisation mécanique 220 ou des moyens de solidarisation magnétique, mentionnés juste ci-dessus, le boîtier 210 peut être prévu pour être solidarisé au train de tiges 110 ou au guide 160, voire au marteau 150. Plus généralement, la localisation du dispositif de mesure 200 sur le pénétromètre dynamique 100 n'est pas limitative du moment que cette localisation permet d'induire une accélération substantielle du dispositif de mesure 200 lorsqu'un impact est appliqué à l'enclume 120 par le marteau 150.
- Dans l'exemple considéré sur les figures, le pénétromètre dynamique 100 peut être qualifié de pénétromètre dynamique léger, dans le sens où le marteau 150 peut être dimensionné pour être soulevé à la main de sa position basse à sa position haute par un opérateur humain. Toutefois, en variante non représentée, le pénétromètre dynamique du système de mesure 1 peut être un pénétromètre dynamique plus lourd, ayant en particulier un marteau dont le poids est plus élevé et dont le soulèvement nécessite un appareillage ad hoc. Plus généralement, le dispositif de mesure 200 est associable à divers types de pénétromètre dynamique.

- Le nombre d'éléments de marteau du marteau 150, dont au moins un est amovible par rapport au reste du marteau, n'est pas limité à trois comme dans l'exemple envisagé sur les figures. Ainsi, en variante non représentée, le marteau 150 peut ne comporter que deux éléments de marteau ou davantage que trois éléments de marteau. Dans tous les cas, le ou chaque élément de marteau du marteau, qui est amovible par rapport au reste du pénétromètre dynamique, est avantageusement vissable directement sur un autre élément de marteau du marteau, comme c'est le cas pour chacun des éléments de marteau 152 et 153 vis-à-vis de l'élément de marteau 151.
- En variante non représentée, le pénétromètre dynamique 100 peut être dépourvu d'un adaptateur tel que l'adaptateur 130 décrit plus haut. Dans ce cas, l'extrémité supérieure du train de tiges 110 coopère directement avec le côté inférieur de l'enclume 120.

## Revendications

1. Dispositif de mesure (200) pour la caractérisation géomécanique d'un sol, comportant :
- un boîtier (210) qui est adapté pour être solidarisé à un pénétromètre dynamique (100),
- une batterie d'alimentation électrique (250), qui est logée dans le boîtier, et
- un système électronique (240), qui est logé dans le boîtier, qui est relié à la batterie d'alimentation électrique, et qui comprend :
- un accéléromètre (242) adapté pour mesurer l'accélération du dispositif de mesure (200),
- un capteur de distance (243), qui est orienté vers un sol (2) lorsque le boîtier est solidarisé au pénétromètre dynamique et qui est adapté pour déterminer la distance entre le dispositif de mesure et la surface (2A) du sol en mesurant le temps nécessaire à une onde émise par le capteur de distance pour effectuer un trajet aller-retour entre le capteur de distance et la surface du sol, et
- une unité de traitement (244), qui est reliée à l'accéléromètre et au capteur de distance et qui, lorsque le système électronique est dans un état de fonctionnement dans lequel la batterie d'alimentation électrique alimente tout le système électronique, est adaptée pour appliquer un premier traitement à des signaux de mesure provenant de l'accéléromètre et du capteur de distance.

2. Dispositif de mesure suivant la revendication 1, dans lequel l'unité de traitement (244) est également adaptée pour :
- passer le système électronique (240) d'un état de veille, dans lequel l'unité de traitement interrompt l'alimentation électrique du capteur de distance (243) par la batterie d'alimentation électrique (250), à l'état de fonctionnement lorsque l'amplitude de l'accélération mesurée par l'accéléromètre (242) dépasse un seuil prédéterminé, et
- passer le système électronique de l'état de fonctionnement à l'état de veille lorsque l'amplitude de l'accélération mesurée par l'accéléromètre reste inférieure audit seuil pendant une durée prédéterminée.

3. Dispositif de mesure suivant l'une des revendications 1 ou 2, dans lequel le dispositif de mesure (200) comporte en outre au moins un amortisseur (230, 260), qui est :
- interposé entre le boîtier (210) et le pénétromètre dynamique (100) lorsque le boîtier est solidarisé au pénétromètre dynamique, et/ou
- interposé entre le boîtier et le système électronique (240).

4. Dispositif de mesure suivant l'une quelconque des revendications précédentes, dans lequel le dispositif de mesure comporte en outre des moyens de solidarisation (220) qui sont adaptés pour solidariser le boîtier (210) au pénétromètre dynamique (100) par une liaison mécanique réversible et/ou par une liaison magnétique.

5. Dispositif de mesure suivant l'une quelconque des revendications précédentes, dans lequel le système électronique (240) comprend également un capteur d'inclinaison (246), qui est relié à l'unité de traitement (244) et qui est adapté pour mesurer l'inclinaison du dispositif de mesure (200) par rapport à la verticale.

6. Système de mesure (1) pour la caractérisation géomécanique d'un sol, comportant :
- un pénétromètre dynamique (100), comprenant un train de tiges (110), prévu pour être enfoncé dans un sol (2), et une enclume (120), prévue pour recevoir des impacts successifs et les transmettre au train de tiges, et
- un dispositif de mesure (200), qui est conforme à l'une quelconque des revendications précédentes et qui est solidarisé au pénétromètre dynamique, en particulier à l'enclume.

7. Système de mesure suivant la revendication 6, dans lequel l'unité de traitement (244) du dispositif de mesure (200) est adaptée, via le premier traitement, pour détecter les différents impacts successifs à partir des signaux de mesure provenant de l'accéléromètre (242) et pour déterminer l'enfoncement du train de tiges (110) dans le sol (2) à l'issue de chaque impact à partir des signaux de mesure provenant du capteur de distance (243).

8. Système de mesure suivant la revendication 6,
dans lequel le système de mesure (1) comprend en outre un dispositif de traitement (300), qui est séparé du pénétromètre dynamique (100) et du dispositif de mesure (200) et qui est adapté pour appliquer un second traitement à des données qui résultent du premier traitement appliqué par l'unité de traitement (244) du dispositif de mesure à des signaux de mesure provenant de l'accéléromètre (242) et du capteur de distance (243) lors des impacts, et
dans lequel l'unité de traitement (244) et le dispositif de traitement (300) sont adaptés, via les premier et second traitements, pour détecter les différents impacts successifs à partir des signaux de mesure provenant de l'accéléromètre et pour déterminer l'enfoncement du train de tiges (110) dans le sol (2) à l'issue de chaque impact à partir des signaux de mesure provenant du capteur de distance.

9. Système de mesure suivant l'une quelconque des revendications 6 à 8, dans lequel le pénétromètre dynamique (100) comporte également un marteau (150) qui :
- est monté mobile sur un guide (160) solidaire de l'enclume (120) entre une position haute, dans laquelle le marteau est écarté de l'enclume, et une position basse, dans laquelle le marteau est au contact de l'enclume, de manière que le marteau applique un impact à l'enclume lorsqu'il passe de la position haute à la position basse, et
- inclut plusieurs éléments de marteau distincts (151, 152, 153) dont au moins un est amovible par rapport au reste du pénétromètre dynamique (100), et
dans lequel l'unité de traitement (244) du dispositif de mesure (200) ou bien l'unité de traitement (244) et le dispositif de traitement (300) sont adaptés, via, respectivement, le premier traitement ou bien les premier et second traitements, pour déterminer celui ou ceux desdits éléments de marteau (151, 152, 153) qui sont effectivement présents dans le marteau (150) à chaque impact, à partir des signaux de mesure provenant de l'accéléromètre (242).

10. Système de mesure suivant la revendication 9, dans lequel le ou chaque élément de marteau (151, 152, 153) du marteau (150), qui est amovible par rapport au reste du pénétromètre dynamique (100), est vissable directement sur un autre élément de marteau du marteau.

11. Système de mesure suivant l'une quelconque des revendications 6 à 10, dans lequel le pénétromètre dynamique (100) comporte un adaptateur de jonction mécanique (130) entre l'enclume (120) et le train de tiges (110), lequel adaptateur de jonction mécanique présente :
- sur un premier côté, une première face d'appui (131) pour l'enclume et un taraudage (132) de réception d'une extrémité filetée du train de tiges (110), le taraudage débouchant sur la première face d'appui, et
- sur un second côté opposé au premier côté, une seconde face d'appui (133) pour l'enclume et un alésage (134) de réception d'une extrémité cylindrique lisse du train de tiges, l'alésage débouchant sur la seconde face d'appui.

12. Procédé de mesure pour la caractérisation géomécanique d'un sol, dans lequel sont utilisés :
- un pénétromètre dynamique (100) comportant un train de tiges (110), prévu pour être enfoncé dans un sol (2), et une enclume (120), prévue pour recevoir des impacts successifs et les transmettre au train de tiges, et
- un dispositif de mesure (200), qui est conforme à l'une quelconque des revendications 1 à 5 et qui est solidarisé au pénétromètre dynamique, en particulier à l'enclume,
et dans lequel le procédé de mesure comprend des étapes consistant à :
- mesurer l'accélération du dispositif de mesure (200) lors de chaque impact, en utilisant l'accéléromètre (242),
- détecter les différents impacts successifs à partir des accélérations mesurées, moyennant le traitement de ces dernières par l'unité de traitement (244),
- mesurer la distance entre le dispositif de mesure (200) et le sol (2) à l'issue de chaque impact, en utilisant le capteur de distance (243), et
- déterminer l'enfoncement du train de tiges (110) dans le sol (2) à l'issue de chaque impact à partir des distances mesurées, moyennant le traitement de ces dernières par l'unité de traitement (244).

13. Procédé de mesure suivant la revendication 12,
dans lequel le pénétromètre dynamique (100) comporte en outre un marteau (150) qui :
- est monté mobile sur un guide (160) solidaire de l'enclume (120),
- est utilisé pour appliquer chaque impact à l'enclume en soulevant le marteau à une hauteur prédéterminée par rapport à l'enclume puis en relâchant le marteau pour qu'il tombe sur l'enclume, et
- inclut plusieurs éléments de marteau distincts (151, 152, 153) dont au moins un est amovible par rapport au reste du pénétromètre dynamique, et
dans lequel le procédé de mesure comprend également une étape consistant à déterminer celui ou ceux desdits éléments de marteau (151, 152, 153) qui sont effectivement présents dans le marteau (150) à chaque impact, à partir de l'accélération mesurée lors de l'impact.

## Patentansprüche

1. Messvorrichtung (200) zur geomechanischen Charakterisierung eines Bodens, die aufweist:
- ein Gehäuse (210), das zur festen Verbindung mit einem dynamischen Penetrometer (100) geeignet ist,
- eine Batterie für die elektrische Versorgung (250), die in dem Gehäuse untergebracht ist, und
- ein elektronisches System (240), das in dem Gehäuse untergebracht ist, das mit der Batterie zur elektrischen Versorgung verbunden ist, und das umfasst:
- einen Beschleunigungsmesser (242), der zum Messen der Beschleunigung der Messvorrichtung (200) geeignet ist,
- einen Abstandssensor (243), der zu einem Boden (2) gerichtet ist, wenn das Gehäuse mit dem dynamischen Penetrometer fest verbunden ist und der durch Messen der Zeit, die für eine vom Abstandssensor gesendete Welle notwendig ist, um den Hin- und Rückweg zwischen dem Abstandssensor und der Oberfläche des Bodens zurückzulegen, zur Bestimmung des Abstands zwischen der Messvorrichtung und der Oberfläche (2A) des Bodens geeignet ist, und
- eine Verarbeitungseinheit (244), die mit dem Beschleunigungsmesser und mit dem Abstandssensor verbunden ist und die, wenn das elektronische System in einem Betriebszustand ist, in dem die Batterie zur elektrischen Versorgung das gesamte elektronische System versorgt, geeignet ist, eine erste Verarbeitung auf Messsignale anzuwenden, die vom Beschleunigungsmesser und vom Abstandssensor kommen.

2. Messvorrichtung nach Anspruch 1, wobei die Verarbeitungseinheit (244) ebenfalls geeignet ist, um:
- das elektronische System (240) aus einem Bereitschaftszustand, in dem die Verarbeitungseinheit die elektrische Versorgung des Abstandssensors (243) durch die Batterie zur elektrischen Versorgung (250) unterbricht, in den Betriebszustand zu versetzen, wenn die Amplitude der vom Beschleunigungsmesser (242) gemessenen Beschleunigung über einem vorher festgelegten Grenzwert liegt, und
- das elektronische System aus dem Betriebszustand in den Bereitschaftszustand zu versetzen, wenn die Amplitude der vom Beschleunigungsmesser (242) gemessenen Beschleunigung während einer vorher festgelegten Zeitdauer unter dem Grenzwert bleibt.

3. Messvorrichtung nach einem der Ansprüche 1 oder 2, wobei die Messvorrichtung (200) ferner mindestens einen Dämpfer (230, 260) aufweist, der:
- zwischen dem Gehäuse (210) und dem dynamischen Penetrometer (100) angeordnet ist, wenn das Gehäuse mit dem dynamischen Penetrometer fest verbunden ist, und/oder
- zwischen dem Gehäuse und dem elektronischen System (240) angeordnet ist.

4. Messvorrichtung nach einem der vorangehenden Ansprüche, wobei die Messvorrichtung ferner Mittel zur festen Verbindung (220) aufweist, die zur festen Verbindung des Gehäuses (210) mit dem dynamischen Penetrometer (100) durch eine reversible mechanische Verbindung und/oder durch eine Magnetverbindung geeignet sind.

5. Messvorrichtung nach einem der vorangehenden Ansprüche, wobei das elektronische System (240) ebenfalls einen Neigungssensor (246) aufweist, der mit der Verarbeitungseinheit (244) verbunden ist und der zur Messung der Neigung der Messvorrichtung (200) im Verhältnis zur Vertikalen geeignet ist.

6. Messsystem (1) zur geomechanischen Charakterisierung eines Bodens, die aufweist:
- ein dynamisches Penetrometer (100), das ein Gestänge (110) umfasst, das zum Eindringen in einen Boden (2) vorgesehen ist, und einen Amboss (120), der zum Empfangen der aufeinanderfolgenden Schläge und ihre Übertragung an das Gestänge vorgesehen ist, und
- eine Messvorrichtung (200) nach einem der vorangehenden Ansprüche und die mit dem dynamischen Penetrometer, vor allem mit dem Amboss, fest verbunden ist.

7. Messsystem nach Anspruch 6, wobei die Verarbeitungseinheit (244) der Messvorrichtung (200) über die erste Verarbeitung zur Ermittlung der verschiedenen aufeinanderfolgenden Schläge ausgehend von den vom Beschleunigungsmesser (242) kommenden Messsignalen und zur Bestimmung des Eindringens des Gestänges (110) in den Boden (2) nach jedem Schlag ausgehend von den vom Abstandssensor (243) kommenden Messsignalen geeignet ist.

8. Messsystem nach Anspruch 6,
wobei das Messsystem (1) ferner eine Verarbeitungsvorrichtung (300) umfasst, die vom dynamischen Penetrometer (100) und von der Messvorrichtung (200) getrennt ist und die zur Anwendung einer zweiten Verarbeitung auf Daten geeignet ist, die aus der ersten Verarbeitung resultieren, die von der Verarbeitungseinheit (244) der Messvorrichtung auf Messsignale angewendet wurde, die vom Beschleunigungsmesser (242) und vom Abstandssensor (243) bei Schlägen kommen, und
wobei die Verarbeitungseinheit (244) und die Verarbeitungsvorrichtung (300) über die erste und zweite Verarbeitung zur Ermittlung der verschiedenen aufeinanderfolgenden Schläge ausgehend von den vom Beschleunigungsmesser kommenden Messsignalen und zur Bestimmung des Eindringens des Gestänges (110) in den Boden (2) nach jedem Schlag ausgehend von den vom Abstandssensor kommenden Messsignalen geeignet sind.

9. Messsystem nach einem der Ansprüche 6 bis 8,
wobei das dynamische Penetrometer (100) ebenfalls einen Hammer (150) aufweist, der:
- auf einer mit dem Amboss (120) fest verbundenen Führung (160) zwischen einer hohen Position, in welcher der Hammer vom Amboss beabstandet ist, und einer niedrigen Position, in welcher der Hammer mit dem Amboss im Kontakt ist, derart beweglich angebracht ist, dass der Hammer einen Schlag ausübt, wenn er aus der hohen Position in die niedrige Position wechselt, und
- mehrere unterschiedliche Hammerelemente (151, 152, 153) einschließt, von denen mindestens eins im Verhältnis zum Rest des dynamischen Penetrometers (100) lösbar ist, und
wobei die Verarbeitungseinheit (244) der Messvorrichtung (200) oder auch die Verarbeitungseinheit (244) und die Verarbeitungsvorrichtung (300) über jeweils die erste Verarbeitung oder auch die erste und zweite Verarbeitung ausgehend von den vom Beschleunigungsmesser (242) kommenden Messsignalen zur Bestimmung desjenigen oder derjenigen der Hammerelemente (151, 152, 153) geeignet sind, die tatsächlich im Hammer (150) bei jedem Schlag vorhanden sind.

10. Messsystem nach Anspruch 9, wobei das oder jedes Hammerelement (151, 152, 153) des Hammers (150), das im Verhältnis zum Rest des dynamischen Penetrometers (100) lösbar ist, direkt auf ein anderes Hammerelement des Hammers aufschraubbar ist.

11. Messsystem nach einem der Ansprüche 6 bis 10, wobei das dynamische Penetrometer (100) einen Adapter für die mechanische Verbindung (130) zwischen dem Amboss (120) und dem Gestänge (110) aufweist, wobei der Adapter für die mechanische Verbindung aufweist:
- auf einer ersten Seite eine erste Abstützfläche (131) für den Amboss und ein Gewinde (132) zur Aufnahme eines gewindeten Endes des Gestänges (110), wobei das Gewinde auf der ersten Abstützfläche ausmündet, und
- auf einer zweiten Seite gegenüber der ersten Seite eine zweite Abstützfläche (133) für den Amboss und eine Bohrung (134) zur Aufnahme eines glatten zylindrischen Endes des Gestänges, wobei die Bohrung auf der zweiten Abstützfläche ausmündet.

12. Verfahren zur Messung der geomechanischen Charakterisierung eines Bodens, wobei verwendet werden:
- ein dynamisches Penetrometer (100), das ein Gestänge (110) aufweist, das zum Eindringen in einen Boden (2) vorgesehen ist, und einen Amboss (120), der zum Empfangen aufeinanderfolgender Schläge und deren Übertragung auf das Gestänge vorgesehen ist, und
- eine Messvorrichtung (200) nach einem der Ansprüche 1 bis 5 und die mit dem dynamischen Penetrometer, vor allem mit dem Amboss, fest verbunden ist,
und wobei das Messverfahren die folgenden Schritte umfasst:
- Messen der Beschleunigung der Messvorrichtung (200) bei jedem Schlag bei Verwendung des Beschleunigungsmessers (242),
- Ermitteln der verschiedenen aufeinanderfolgenden Schläge ausgehend von den gemessenen Beschleunigungen mittels der Verarbeitung derselben durch die Verarbeitungseinheit (244),
- Messen des Abstands zwischen der Messvorrichtung (200) und dem Boden (2) nach jedem Schlag bei Verwendung des Abstandssensors (243), und
- Bestimmen des Eindringens des Gestänges (110) in den Boden (2) nach jedem Schlag ausgehend von den gemessenen Abständen mittels der Verarbeitung derselben durch die Verarbeitungseinheit (244).

13. Messverfahren nach Anspruch 12,
wobei das dynamische Penetrometer (100) ferner einen Hammer (150) aufweist, der:
- auf einer mit dem Amboss (120) fest verbundenen Führung (160) beweglich angebracht ist,
- verwendet wird, um jeden Schlag auf den Amboss durch Anheben des Hammers in eine vorher festgelegte Höhe im Verhältnis zum Amboss und dann durch Loslassen des Hammers, damit er auf den Amboss fällt, anzuwenden, und
- mehrere unterschiedliche Hammerelemente (151, 152, 153) einschließt, von denen mindestens eins im Verhältnis zum Rest des dynamischen Penetrometers lösbar ist, und
wobei das Messverfahren ebenfalls einen Schritt umfasst, der darin besteht, ausgehend von der beim Schlag gemessenen Beschleunigung das oder die der Hammerelemente (151, 152, 153) zu bestimmen, die tatsächlich bei jedem Schlag im Hammer (150) vorhanden sind.

## Claims

1. A measuring device (200) for the geomechanical characterization of a soil, comprising:
- a housing (210) which is adapted to be secured to a dynamic penetrometer (100),
- a power supply battery (250), which is accommodated in the casing, and
- an electronic system (240), which is accommodated in the casing, which is connected to the power supply battery, and which comprises:
- an accelerometer (242) adapted to measure an acceleration of the measuring device (200),
- a distance sensor (243), which is oriented towards a soil (2) when the housing is secured to the dynamic penetrometer and which is adapted to determine a distance between the measuring device and a surface (2A) of the soil by measuring the time required for a wave emitted by the distance sensor to travel back and forth between the distance sensor and the surface of the soil, and
- a processing unit (244), which is connected to the accelerometer and the distance sensor and which, when the electronic system is in an operating state in which the power supply battery powers entirely the electronic system, is adapted to apply a first processing to measuring signals coming from the accelerometer and the distance sensor.

2. The measuring device according to claim 1, wherein the processing unit (244) is also adapted to:
- switch the electronic system (240) from a standby state, in which the processing unit interrupts the power supply to the distance sensor (243) by the power supply battery (250), to the operating state when an amplitude of the acceleration measured by the accelerometer (242) exceeds a predetermined threshold, and
- switch the electronic system from the operating state to the standby state when the amplitude of the acceleration measured by the accelerometer remains below said threshold for a predetermined time.

3. The measuring device according to one of claims 1 or 2, wherein the measuring device (200) further comprises at least one damper (230, 260), which is:
- interposed between the housing (210) and the dynamic penetrometer (100) when the housing is secured to the dynamic penetrometer, and/or
- interposed between the housing and the electronic system (240).

4. The measuring device according to any one of the preceding claims, in which the measuring device further comprises a fastening (220) adapted to secure the housing (210) to the dynamic penetrometer (100) by a reversible mechanical connection and/or by a magnetic connection.

5. The measuring device according to any of the preceding claims, wherein the electronic system (240) also comprises a tilt sensor (246), which is connected to the processing unit (244) and which is adapted to measure a tilt of the measuring device (200) relative to the vertical.

6. A measuring system (1) for the geomechanical characterization of a soil, comprising:
- a dynamic penetrometer (100), comprising a train of rods (110), designed to be driven into the soil (2), and an anvil (120), designed to receive successive impacts and transmit them to the train of rods, and
- a measuring device (200), which is according to any of the preceding claims and which is secured to the dynamic penetrometer, in particular to the anvil.

7. The measuring system according to claim 6, wherein the processing unit (244) of the measuring device (200) is adapted, via the first processing, to detect individually the successive impacts from the measuring signals coming from the accelerometer (242) and to determine boring of the train of rods (110) into the soil (2) after each impact from the measuring signals from the distance sensor (243).

8. The measuring system according to claim 6,
wherein the measuring device (1) further comprises a processing device (300), which is separate from the dynamic penetrometer (100) and the measuring device (200) and which is adapted to apply a second processing to data which result from the first processing applied by the processing unit (244) of the measuring device to measuring signals from the accelerometer (242) and the distance sensor (243) during impacts, and
in which the processing unit (244) and the processing device (300) are adapted, via the first and second processes, to detect individually the successive impacts coming from the measuring signals coming from the accelerometer and to determine boring of the train of rods (110) into the soil (2) after each impact from the measuring signals coming from the distance sensor.

9. The measuring system according to any of claims 6 to 8,
wherein the dynamic penetrometer (100) also comprises a hammer (150) that:
- is movably mounted on a guide (160) attached to the anvil (120) between a high position, in which the hammer is moved away from the anvil, and a low position, in which the hammer is in contact with the anvil, so that the hammer applies an impact to the anvil when the hammer passes from the high position to the low position, and
- includes several separate hammer elements (151, 152, 153), at least one of which is removable from the rest of the dynamic penetrometer (100), and
wherein either the processing unit (244) of the measuring device (200) or the processing unit (244) and the processing device (300) are adapted, via, respectively, either the first treatment or the first and second treatments, to determine which of said hammer elements (151, 152, 153) is or are actually present in the hammer (150) at each impact, from the measuring signals coming from the accelerometer (242).

10. The measuring system according to claim 9, wherein the or each hammer element (151, 152, 153) of the hammer (150), which is removable relative to the rest of the dynamic penetrometer (100), is screwable directly onto another hammer element of the hammer.

11. The system device according to any one of claims 6 to 10, wherein the dynamic penetrometer (100) has a mechanical connection adapter (130) between the anvil (120) and the train of rods (110), whose mechanical connection adapter has:
- on a first side, a first bearing face (131) for the anvil and an internal thread (132) for receiving a threaded end of the train of rods (110), the internal thread opening to the first bearing face, and
- on a second side opposite the first side, a second bearing face (133) for the anvil and a bore (134) for receiving a smooth cylindrical end of the train of rods, the bore opening onto the second bearing face.

12. A measuring method for the geomechanical characterization of a soil,
wherein the measuring method uses:
- a dynamic penetrometer (100) comprising a train of rods (110), designed to be driven into the soil (2), and an anvil (120), designed to receive successive impacts and transmit them to the train of rods, and
- a measuring device (200), which is according to any one of claims 1 to 5 and which is secured to the dynamic penetrometer, in particular to the anvil,
and wherein the measuring method comprises the steps of:
- measuring an acceleration of the measuring device (200) upon each impact, using the accelerometer (242),
- detecting individually the successive impacts from the measured accelerations, by processing them by the processing unit (244),
- measuring a distance between the measuring device (200) and the soil (2) after each impact, using the distance sensor (243), and
- determining boring of the train of rods (110) into the soil (2) after each impact from the measured distances, by processing them with the processing unit (244).

13. The measuring method according to claim 12,
wherein the dynamic penetrometer (100) further comprises a hammer (150) which:
- is movably mounted on a guide (160) attached to the anvil (120),
- is used to apply each impact to the anvil by raising the hammer to a predetermined height from the anvil and then releasing the hammer to fall on the anvil, and
- includes several separate hammer elements (151, 152, 153), at least one of which is removable from the rest of the dynamic penetrometer, and
wherein the measuring method also comprises a step of determining which of said hammer elements (151, 152, 153) is or are actually present in the hammer (150) at each impact, from the measured acceleration upon the impact.
